# EUROPEAN PATENT APPLICATION

(11) **EP 4 834 338 A1**
(43) Date of publication of application: **04.11.2026**
(21) Application number: 24913192.1
(22) Date of filing: 26.04.2024
(51) Int. Cl.: A61K 31/137, A61K 31/513, A61K 9/00, A61P 35/00

(54) **PHARMACEUTICAL COMPOSITION FOR DISRUPTION OF TUMOR BLOOD VESSELS**

(30) Priority: 26.12.2023 KR 20230190961
(71) Applicant: Endocura Inc., Seoul 03181 (KR); Samsung Medical Foundation, Seoul 03181 (KR)
(72) Inventor: PARK, Jung Ho, Seoul 03165 (KR)
(74) Representative: Wichmann, Hendrik
(86) International application number: PCT/KR2024/005715
(87) International publication number: WO 2025/143377

(57) **Abstract**

According to an exemplary embodiment, a pharmaceutical composition for tumor vascular disruption may comprise alkaline 5-fluorouracil (5-FU) and epinephrine. Epinephrine may exert a synergistic effect with the tumor vascular endothelial cell-killing effect of alkaline 5-fluorouracil, thereby improving anticancer efficacy.

## Description

### TECHNICAL FIELD

The present invention relates to a pharmaceutical composition for tumor vascular disruption.

### BACKGROUND ART

5-Fluorouracil (5-FU) is an anticancer chemotherapeutic agent widely used for cancer treatment. 5-Fluorouracil is an analog of uracil present in the human body, and exhibits anticancer effects by inhibiting DNA synthesis and repair through inhibition of thymidylate synthase, and by inhibiting RNA function.

Since 5-fluorouracil is mainly administered intravenously and thus must be directly administered in a hospital, and also has side effects such as thrombosis, oral 5-FU prodrugs have recently been developed.

5-Fluorouracil has significant effects, but may cause myelosuppression or gastrointestinal toxicity due to phosphorylation in the gastrointestinal tract. In addition, cardiotoxicity, which is a serious side effect that may be life-threatening, has been reported, for example, at rates of up to 1.5% to 18%.

Accordingly, active research has been conducted to reduce the side effects of 5-fluorouracil while maintaining its anticancer efficacy. For example, Korean Patent Publication No.10-2016-0049312 seeks to effectively ameliorate side effects of anticancer agents, such as inflammatory responses, intestinal damage, and myelosuppression caused by 5-fluorouracil, through the use of cyanidin-3-glucoside.

### DISCLOSURE

### TECHNICAL PROBLEM

An object of the present invention is to provide a pharmaceutical composition for tumor vascular disruption having excellent anticancer efficacy and reduced side effects.

### TECHNICAL SOLUTION

A pharmaceutical composition for tumor vascular disruption according to an exemplary embodiment may include alkaline 5-fluorouracil (5-FU) and epinephrine.

In one embodiment, the alkalinity may have a pH of 8 to 9.

In one embodiment, the alkaline 5-fluorouracil may be in a form in which 5-fluorouracil is dissolved in an alkaline solvent.

In one embodiment, the alkaline 5-fluorouracil may cause death of vascular endothelial cells by increasing expression of thrombospondin-1 protein in tumor vascular endothelial cells.

In one embodiment, the concentration of the alkaline 5-fluorouracil may be 0.1 to 600 mM based on the total composition.

In one embodiment, the epinephrine may induce apoptosis of tumor vascular endothelial cells.

In one embodiment, the concentration of the epinephrine may be 0.001 to 0.2 mg/mL based on the total composition.

In one embodiment, the composition may further comprise one or more selected from glucose, a nitric oxide production inhibitor, bevacizumab, capric acid or a physiologically acceptable salt thereof, and poloxamer.

In one embodiment, the nitric oxide production inhibitor may comprise one or more selected from L-NMMA (N^{G}-Methyl-L-arginine), L-NAME (N^{G}-Nitro-L-arginine methyl ester), L-NA (Nitro Arginine), and 7NI (Nitroindazole).

In one embodiment, the composition may further comprise one or more selected from cisplatin, carboplatin, cyclophosphamide, paclitaxel, tamoxifen, toremifene, fulvestrant, diindolylmethane, exemestane, raloxifene, an aromatase inhibitor, doxorubicin, oxaliplatin, vincristine, gemcitabine, an anthracycline, a taxane, irinotecan, docetaxel, and eribulin.

In one embodiment, the cancer may comprise a solid cancer.

In one embodiment, the composition may be a sustained-release formulation.

In one embodiment, the composition may be administered by local injection around a tumor.

In one embodiment, the area around the tumor may be a submucosal layer in which tumor blood vessels are located.

A local administration agent for a solid cancer according to an exemplary embodiment may comprise the pharmaceutical composition for tumor vascular disruption.

### ADVANTAGEOUS EFFECTS OF INVENTION

The pharmaceutical composition for tumor vascular disruption according to the present invention may comprise alkaline 5-fluorouracil and epinephrine.

For example, when the alkaline 5-fluorouracil is injected into the base of a tumor, the alkaline 5-fluorouracil may induce apoptosis of tumor vascular endothelial cells, thereby destroying the tumor. In addition, the alkaline 5-fluorouracil may inhibit tumor angiogenesis.

For example, when epinephrine is injected into the base of a tumor, the epinephrine may induce apoptosis of tumor vascular endothelial cells. The epinephrine may exert a synergistic effect with the tumor vascular endothelial cell death effect of alkaline 5-fluorouracil, thereby enhancing an anticancer effect. In addition, the epinephrine may suppress bleeding by constricting blood vessels at the epinephrine injection site. Accordingly, the epinephrine may help the death of tumor vascular endothelial cells by suppressing systemic absorption of 5-fluorouracil while reducing side effects and maintaining efficacy.

Since the pharmaceutical composition for disrupting tumor blood vessels according to the present invention destroys blood vessels supplying blood to a tumor rather than the tumor itself, the pharmaceutical composition may be applied to various solid cancers.

### BRIEF DESCRIPTION OF THE DRAWINGS

FIG. 1 illustrates an example in which a pharmaceutical composition for tumor vascular disruption according to one embodiment is administered by local injection around a tumor.
FIGS. 2a and 2b show results of confirming the HUVEC cell death effect according to epinephrine concentration using MTS.
FIGS. 3a and 3b show results of confirming the HUVEC cell death effect according to epinephrine concentration using Western blot.
FIG. 4 shows results of confirming DNA laddering by gel electrophoresis according to epinephrine concentration.
FIGS. 5a to 5e show results of confirming the HUVEC cell death effect according to epinephrine concentration using confocal fluorescence microscopy.
FIG. 6a is a graph showing tumor size (volume) when 5-FU is administered or when 5-FU and epinephrine are administered together after xenografting human gastric cancer cells into nude mice.
FIG. 6b is a photograph showing tumor size (volume) when 5-FU and epinephrine are administered together after xenografting human gastric cancer cells into nude mice.
FIG. 7a is a graph showing tumor size (volume) when 5-FU and epinephrine are administered together and when 5-FU, epinephrine, and L-glucose are administered together after xenografting human gastric cancer cells into nude mice.
FIG. 7b is a photograph showing tumor size (volume) when 5-FU, epinephrine, and L-glucose are administered together after xenografting human gastric cancer cells into nude mice.

### DETAILED DESCRIPTION OF THE EMBODIMENTS

A pharmaceutical composition for tumor vascular disruption according to an exemplary embodiment (hereinafter, referred to as a "composition") comprises alkaline 5-fluorouracil (5-FU) and epinephrine. For example, the composition may comprise epinephrine to induce death of tumor vascular endothelial cells, thereby exerting a synergistic effect with the tumor vascular endothelial cell death effect of the alkaline 5-fluorouracil and significantly enhancing an anticancer effect.

Hereinafter, the composition according to exemplary embodiments of the present invention will be described in detail with reference to the drawings and Examples. However, the drawings and Examples are merely exemplary, and the present invention is not limited thereto.

According to an exemplary embodiment, the composition may comprise alkaline 5-fluorouracil (5-FU) and epinephrine.

Alkaline 5-fluorouracil exerts an anticancer effect by destroying tumor blood vessels and inhibiting angiogenesis, thereby blocking or suppressing the supply of nutrients or oxygen to cancer cells.

In one embodiment, the alkaline 5-fluorouracil may cause death of vascular endothelial cells by increasing expression of thrombospondin-1 (TSP-1) protein in tumor vascular endothelial cells. Because this is a result of destruction of blood vessels supplying blood to a tumor rather than destruction of the tumor itself, the alkaline 5-fluorouracil may be applied to various solid cancers.

When 5-fluorouracil is mixed with an alkaline solvent, its anticancer efficacy may be enhanced. For example, it may provide superior anticancer efficacy when mixed with an alkaline solvent as compared to a neutral solvent or an acidic solvent. For example, alkaline 5-fluorouracil may target vascular endothelial cells within cancer tissue and induce apoptosis. For example, when alkaline 5-fluorouracil is injected into the base of a tumor tissue, it may induce apoptosis of vascular endothelial cells supplying blood to the tumor, thereby destroying the blood vessels and causing necrosis of the tumor. In this case, the pH of 5-fluorouracil is an important factor in inducing apoptosis of tumor vascular endothelial cells, and when the pH is weakly alkaline, for example, 8.4 to 9.0, apoptosis of vascular endothelial cells may be significantly increased as compared with neutral or acidic 5-fluorouracil.

In one embodiment, the alkaline 5-fluorouracil may be in a form in which 5-fluorouracil is dissolved in an alkaline solvent. The alkaline solvent may be, for example, alkaline water, physiological saline, or the like.

In one embodiment, the alkalinity may have a pH of 8.0 to 9.0. For example, the pH may be 8.0 to 9.0, 8.2 to 9.0, 8.4 to 9.0, or 8.4 to 8.8. However, the present invention is not limited thereto.

In one embodiment, the concentration of alkaline 5-fluorouracil may be 0.1 mM to 600 mM, 0.1 mM to 500 mM, 0.5 mM to 390 mM, 1 mM to 350 mM, 10 mM to 300 mM, 50 mM to 250 mM, or 100 mM to 200 mM in the total composition. However, the present invention is not limited thereto.

Epinephrine may induce apoptosis of tumor vascular endothelial cells. Accordingly, epinephrine may exert a synergistic effect with the vascular endothelial cell-killing effect of 5-FU, thereby maximizing an anticancer effect. In addition, epinephrine may suppress bleeding by constricting blood vessels at the epinephrine injection site (for example, within subcutaneous tissue or submucosal tissue), and may reduce side effects by suppressing systemic absorption of 5-FU. The duration of effect of 5-FU, whose absorption is suppressed, may be prolonged, thereby improving the vascular endothelial cell-killing effect.

In one embodiment, the concentration of epinephrine may be 0.001 to 0.2 mg/mL based on the total composition. For example, the concentration may be 0.001 to 0.1 mg/mL, and for example, may be 0.002 to 0.2 mg/mL. For example, when 200 mg and 10 cc of alkaline 5-fluorouracil are injected into the submucosal layer of a normal gastric wall immediately adjacent to cancer cells, the amount of epinephrine administered may be 0.001 mg/mL to 0.2 mg/mL.

The epinephrine concentration of 0.1 mg/mL may be expressed as 1:10,000. The ratio of 1:10,000 may refer to epinephrine diluted in a solution (for example, physiological saline) at a ratio of 1:10,000. For example, the concentration of epinephrine suitable for administration may range from 1:10,000 to 1:1,000,000.

In one embodiment, the composition may further comprise one or more selected from glucose, a nitric oxide production inhibitor, bevacizumab, capric acid or a physiologically acceptable salt thereof, and poloxamer.

In some embodiments, the glucose may comprise one or more selected from D-glucose and L-glucose.

For example, a high concentration of D-glucose may delay replication of endothelial cells (replicative delay) and promote apoptosis. For example, when exposed at a high concentration (e.g., 25 mM or more) for 48 hours or longer, DNA fragmentation may increase and expression of thrombospondin-1 may increase, thereby inducing apoptosis of endothelial cells. A high concentration of glucose may increase reactive oxygen species within tumor vascular endothelial cells, thereby increasing oxidative stress. In other words, the increase in reactive oxygen species caused by a high concentration of glucose shares a mechanism with the endothelial cell toxicity caused by alkaline 5-fluorouracil.

For example, L-glucose may induce cell death by suppressing metabolism of tumor vascular endothelial cells.

For example, the glucose may be included in an amount such that the concentration thereof is 10 mM to 500 mM, 20 mM to 250 mM, 25 mM to 150 mM, 30 mM to 120 mM, or 50 mM to 100 mM, based on the total composition.

In one embodiment, the nitric oxide production inhibitor may comprise one or more selected from L-NMMA (N^{G}-Methyl-L-arginine), L-NAME (N^{G}-Nitro-L-arginine methyl ester), L-NA (Nitro Arginine), and 7NI (Nitroindazole).

Nitric oxide (Nitrile Oxide) is synthesized and secreted by NO synthase in vascular endothelial cells, dilates blood vessels, and protects cells by inhibiting vascular endothelial cells from progressing toward apoptosis in response to external stimuli such as lipopolysaccharide (LPS), angiotensin II, caspase-3 overexpression, and TNF-alpha.

A nitric oxide production inhibitor may constrict tumor blood vessels and promote thrombosis within tumor blood vessels damaged by 5-fluorouracil. For example, tumor vascular endothelial cells may be damaged or destroyed by alkaline 5-fluorouracil, thereby causing thrombus formation within the tumor blood vessels. The nitric oxide production inhibitor may promote occlusion of such tumor blood vessels, thereby more effectively inducing necrosis of a tumor mass.

In some embodiments, the nitric oxide production inhibitor may preferably comprise L-NMMA (N^{G}-Methyl-L-arginine).

L-NMMA is a nonspecific NO synthase inhibitor and effectively increases blood pressure when administered into blood vessels. Under physiological conditions, inhibition of NO synthase alone by L-NMMA does not cause platelet activation in vivo. However, when damage occurs to endothelial cells of tumor blood vessels, platelet activation and thrombosis may be induced. In other words, damaged vascular endothelial cells may induce thrombus formation. Accordingly, when L-NMMA is used together with 5-fluorouracil, a tumor vascular endothelial cell-killing effect and tumor vascular occlusion may both be provided, thereby providing excellent anticancer efficacy.

For example, L-NMMA may be included in an amount of 0.5 mg/kg to 20 mg/kg, 1 mg/kg to 10 mg/kg, 2 mg/kg to 8 mg/kg, or 4 mg/kg to 6 mg/kg, based on the weight of a subject to which the drug is administered. However, the present invention is not limited thereto.

As another example, the concentration of the nitric oxide production inhibitor may be 0.1 mM to 300 mM, 0.1 mM to 250 mM, 1 mM to 200 mM, or 10 mM to 200 mM, based on the total composition. However, the present invention is not limited thereto.

Bevacizumab is a recombinant humanized monoclonal antibody for inhibiting vascular endothelial growth factor (VEGF), which promotes angiogenesis. Bevacizumab may generally be used as an anticancer agent alone or in combination with another drug. Conventionally, bevacizumab has been mainly administered by intravenous injection, and for example, in the case of advanced gastric cancer, it is known that when bevacizumab is used in combination with fluoropyrimidine-cisplatin therapy, progression-free survival and overall response rate are improved. However, in some embodiments, bevacizumab may be provided in the composition in a sustained-release form, thereby inhibiting angiogenesis of cancer cells and more effectively eliminating blood vessels distributed within a tumor.

In one embodiment, the composition may be a sustained-release formulation. As the exposure time to tumor vascular endothelial cells increases, the cell-killing effect may be further increased. In order to provide the composition as a sustained-release formulation, the composition may further comprise capric acid or a physiologically acceptable salt thereof, and poloxamer.

Capric acid or a physiologically acceptable salt thereof, and poloxamer have low toxicity, are biocompatible, and exhibit superior in vivo stability. For example, by mixing capric acid or a physiologically acceptable salt thereof, and poloxamer, viscosity may be increased and the sol-gel transition temperature of the poloxamer may be elevated. For example, the sol-gel transition temperature may be between room temperature and body temperature, for example, 20 to 40°C or 25 to 37°C. Within the above range, storage, transportation, and use in a sol state at room temperature are convenient. When acting in vivo, the composition may exist in a gel state, and diffusion within tissue may also be facilitated.

In some embodiments, the weight ratio of capric acid to poloxamer may be 0.25 to 3.4:30, 1 to 3.4:30, or 2 to 3:30, and more specifically, the weight ratio of capric acid to poloxamer may be 2.75 to 3.3:30 or 2.8 to 3.2:30. Within the above range, the sol-gel transition of the carrier may readily occur at a temperature between 25 and 36°C.

Capric acid is a compound represented by Chemical Formula 1 below and is a type of saturated fatty acid.

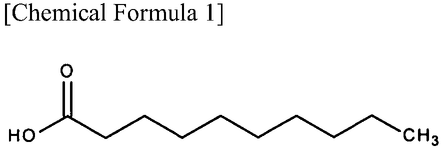

The pharmaceutically acceptable salt may be a salt prepared using capric acid and a relatively non-toxic acid or base. The pharmaceutically acceptable salt may include, for example, a metal salt or an acid addition salt.

The metal salt may be a sodium, potassium, or calcium salt. The metal salt may be prepared using a base. For example, an alkali metal or alkaline earth metal salt may be obtained by dissolving the compound in an excess of an alkali metal hydroxide or alkaline earth metal hydroxide solution, filtering the resulting insoluble compound salt, and evaporating and/or drying the filtrate.

An acid addition salt may be prepared from an inorganic acid such as hydrochloric acid, nitric acid, phosphoric acid, sulfuric acid, hydrobromic acid, hydroiodic acid, nitrous acid, or phosphorous acid, and from a salt of a non-toxic organic acid such as an aliphatic mono- and dicarboxylate, a phenyl-substituted alkanoate, a hydroxy alkanoate, an alkandioate, an aromatic acid, or an aliphatic or aromatic sulfonic acid. These physiologically non-toxic salts may include sulfates, pyrosulfates, bisulfates, sulfites, bisulfites, nitrates, phosphates, monohydrogen phosphates, dihydrogen phosphates, metaphosphates, pyrophosphates, chlorides, bromides, iodides, fluorides, acetates, propionates, decanoates, caprylates, acrylates, formates, isobutyrates, caprates, heptanoates, propylenates, oxalates, malonates, succinates, suberates, sebacates, fumarates, maleates, butene-1,4-dioates, hexane-1,6-dioates, benzoates, chlorobenzoates, methylbenzoates, dinitrobenzoates, hydroxybenzoates, methoxybenzoates, phthalates, terephthalates, benzene sulfonates, toluene sulfonates, chlorobenzene sulfonates, xylene sulfonates, phenylacetates, phenylpropionates, phenylbutyrates, citrates, lactates, β-hydroxybutyrates, glycolates, maleates, tartrates, methanesulfonates, propanesulfonates, naphthalene-1-sulfonates, naphthalene-2-sulfonates, or mandelates. For example, an acid addition salt of the compound represented by Chemical Formula 1 may be prepared by dissolving the compound in an excess aqueous acid solution and precipitating the salt using a water-miscible organic solvent, such as methanol, ethanol, acetone, or acetonitrile.

The poloxamer may be a triblock copolymer comprising a poly(ethylene oxide)-poly(propylene oxide)-poly(ethylene oxide) (PEO-PPO-PEO) structure. For example, the poloxamer may be represented by the following Chemical Formula 2.

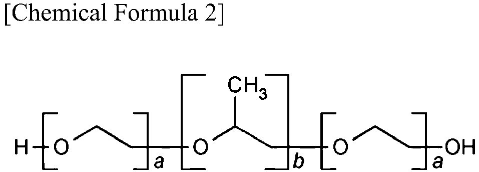

The molecular weight of the poloxamer may be, for example, a weight average molecular weight of 1,000 to 100,000, 10,000 to 100,000, 10,000 to 20,000, or 10,000 to 15,000. The molecular weight may be appropriately selected by a person skilled in the art depending on the material to be delivered.

Poloxamers are generally designated by a numbering system representing the approximate molecular weight of the poloxamer and the percentage content of polyoxyethylene, and are also referred to by the trade name Pluronic^{®}. For example, Poloxamer 407 and the trade name Pluronic F-127 may be used interchangeably.

For example, the poloxamer may be Poloxamer 101, Poloxamer 105, Poloxamer 108, Poloxamer 122, Poloxamer 123, Poloxamer 124, Poloxamer 181, Poloxamer 182, Poloxamer 183, Poloxamer 184, Poloxamer 185, Poloxamer 188, Poloxamer 212, Poloxamer 215, Poloxamer 217, Poloxamer 231, Poloxamer 234, Poloxamer 235, Poloxamer 237, Poloxamer 238, Poloxamer 282, Poloxamer 284, Poloxamer 288, Poloxamer 331, Poloxamer 333, Poloxamer 334, Poloxamer 335, Poloxamer 338, Poloxamer 401, Poloxamer 402, Poloxamer 403, Poloxamer 407, or the like.

The poloxamer is a type of surfactant and may form micelles in an aqueous environment, allowing the loading of substances. A hydrogel composed of poloxamers may form a matrix and may be used as a carrier for the localized and sustained release of substances. The poloxamer hydrogel may include cross-linking between poloxamer molecules. The poloxamer may be used in various molecular weights and ratios.

In one embodiment, the composition may further comprise a known anticancer agent. For example, the known anticancer agent may comprise cisplatin, carboplatin, cyclophosphamide, paclitaxel, tamoxifen, toremifene, fulvestrant, diindolylmethane, exemestane, raloxifene, an aromatase inhibitor, doxorubicin, oxaliplatin, vincristine, gemcitabine, an anthracycline, a taxane, irinotecan, docetaxel, eribulin, or the like. However, the present invention is not limited thereto. The component may further comprise another carrier or the like, may be formulated together with a carrier, and may be appropriately selected by a person skilled in the art in consideration of the type of biologically active substance, the route by which the carrier is administered, and the like. The additional component may further comprise another carrier or the like, may be formulated together with a carrier, and may be appropriately selected by a person skilled in the art in consideration of the type of biologically active substance, the route by which the carrier is administered, and the like.

In some embodiments, the composition may comprise one or more active ingredients having the same or similar functions in connection with the treatment of the tumor.

In some embodiments, the composition may further comprise a compound that maintains or enhances the solubility and/or absorbability of the active ingredient.

In one embodiment, the tumor may comprise a solid cancer.

In some embodiments, the tumor may be, for example, gastric cancer, liver cancer, pancreatic cancer, osteosarcoma, skin cancer, lung cancer, neuroblastoma, uterine cancer, kidney cancer, prostate cancer, breast cancer, colorectal cancer, bile duct cancer, bladder cancer, ovarian cancer, brain tumor, cervical cancer, prostate cancer, testicular cancer, penile cancer, genitourinary tract cancer, seminoma, esophageal cancer, laryngeal cancer, gastrointestinal cancer, keratoacanthoma, follicular carcinoma, melanoma, small-cell lung carcinoma, non-small-cell lung carcinoma (NSCLC), lung adenocarcinoma, squamous cell carcinoma of the lung, colon cancer, thyroid cancer, papillary carcinoma, cholangiocarcinoma, renal cancer, bone cancer, bone marrow disorder, hairy cell carcinoma, oral and pharyngeal (oral) cancer, lip cancer, tongue cancer, oral cavity cancer, salivary gland cancer, pharyngeal cancer, small intestine cancer, colon cancer, rectal cancer, vulvar cancer, thyroid cancer, endometrial cancer, central nervous system cancer, peritoneal cancer, hepatocellular carcinoma, head cancer, and neck cancer, but is not limited thereto.

The formulation of the composition may be prepared as an oral or parenteral formulation. For example, the formulation may be suitable for administration by the oral, rectal, nasal, topical (including buccal and sublingual), subcutaneous, vaginal, or parenteral (including intramuscular, subcutaneous, and intravenous) route. Alternatively, the formulation may be suitable for administration by inhalation or insufflation.

The composition may be in the form of an injectable preparation. Since it does not form precipitates in a biological environment, such as blood, it may be administered even through a thin injection needle. The composition is preferably in the form of an injectable preparation.

In one embodiment, the composition may be administered by local injection around a tumor.

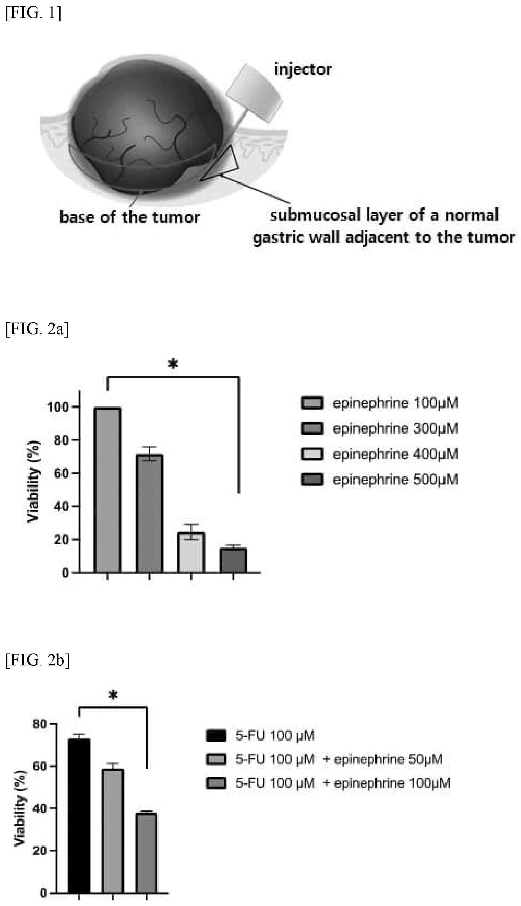

Referring to FIG. 1, for example, the composition may be delivered to the basal layer of a tumor through an injector using an endoscope. Specifically, the composition may be injected into a submucosa zone of a normal gastric wall immediately adjacent to the tumor toward the basal layer of the tumor. An effect of inducing death of vascular endothelial cells or an effect of inhibiting synthesis of neovascular vessels may be exhibited by a drug delivered to vascular endothelial cells of blood vessels supplying blood to the tumor tissue.

The local injection may deliver a greater amount of the drug not only to the submucosal layer around the tumor but also to the surrounding lymph nodes, as compared with conventional intravenous administration of an anticancer agent, and may reduce side effects of the anticancer agent as compared with systemic administration.

The composition according to one embodiment may be administered in a pharmaceutically effective amount. The effective dosage level may be determined according to the type and severity of the patient's disease, the activity of the drug, sensitivity to the drug, time of administration, route of administration, rate of excretion, duration of treatment, factors including drugs used in combination therewith, and other factors well known in the medical field.

A local administration agent for a solid cancer according to an exemplary embodiment may comprise the composition described above. Specific examples of the solid cancer are the same as those described above.

In some embodiments, the composition may be administered as a monotherapy or in combination with another therapeutic agent. The components included in the anticancer combination preparation may be administered sequentially or simultaneously and may be administered in a single dose or in multiple doses. It is important to administer an amount that can achieve the maximum effect with the minimum amount without causing side effects, taking all of the above-described factors into consideration, and such an amount may be readily determined by a person skilled in the art.

For example, the dosage of the composition may vary widely depending on the patient's body weight, age, sex, health condition, diet, time of administration, method of administration, excretion rate, severity of the disease, and the like. An appropriate dosage may vary, for example, depending on the amount of the drug accumulated in the patient's body and/or the specific efficacy of the carrier of the present invention used. For example, the dosage may be 0.01 µg to 1 g per kilogram of body weight and may be administered once or several times per unit period, such as daily, weekly, monthly, or yearly. Alternatively, the composition may be continuously administered over a long period of time using an infusion pump. The number of repeated administrations may be determined in consideration of the residence time of the drug in the body, the concentration of the drug in the body, and the like. Even after treatment has been completed according to the progress of disease treatment, the composition may be administered to prevent recurrence.

Hereinafter, the present invention will be described in detail with reference to Examples in order to specifically illustrate the present invention.

### <Example>

### Example 1: Confirmation of the Apoptosis-Inducing Effect on HUVECs According to Epinephrine Concentration Using an MTS Assay

Cytotoxicity against HUVECs (human umbilical vein endothelial cells) according to the concentration of epinephrine was measured using a CellTiter 96 AQueous One Solution Cell Proliferation Assay. HUVEC cells were seeded (seeding) in a 96-well plate at a density of 5×10³ cells/well and cultured for 24 hours. Thereafter, the cells were cultured with DMEM medium containing various concentrations of epinephrine for 24 hours. The cells were then washed twice with PBS, and 100 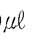 of fresh growth DMEM medium was added, followed by further incubation for 48 hours. After replacing the medium with 100 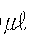 of fresh growth DMEM medium, 20 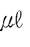 of 3-(4,5-dimethylthiazol-2-yl)-5-(3-carboxymethoxyphenyl)-2-(4-sulfophenyl)-2H-tetrazolium (MTS) was added. After an additional 2 hours of incubation, absorbance was measured at 490 nm using a BIO-RAD Model 680 microplate reader (Spark, TECAN, CA, USA). The results are shown in FIGS. 2a and 2b.

As shown in FIG. 2a, the viability of HUVECs decreased in proportion to the concentration of epinephrine. As shown in FIG. 2b, when 100µm of 5-FU was co-administered, the viability of HUVECs decreased in proportion to the concentration of epinephrine. Accordingly, it can be seen that epinephrine exerts a synergistic effect on 5-FU-induced cell death.

### Example 2: Confirmation of the Apoptosis Mechanism of Epinephrine in HUVECs by Western Blot

Western blot analysis was performed to confirm the expression of FAS, cleaved caspase-3, and cleaved caspase-9, which are activated during epinephrine-induced apoptosis of HUVECs.

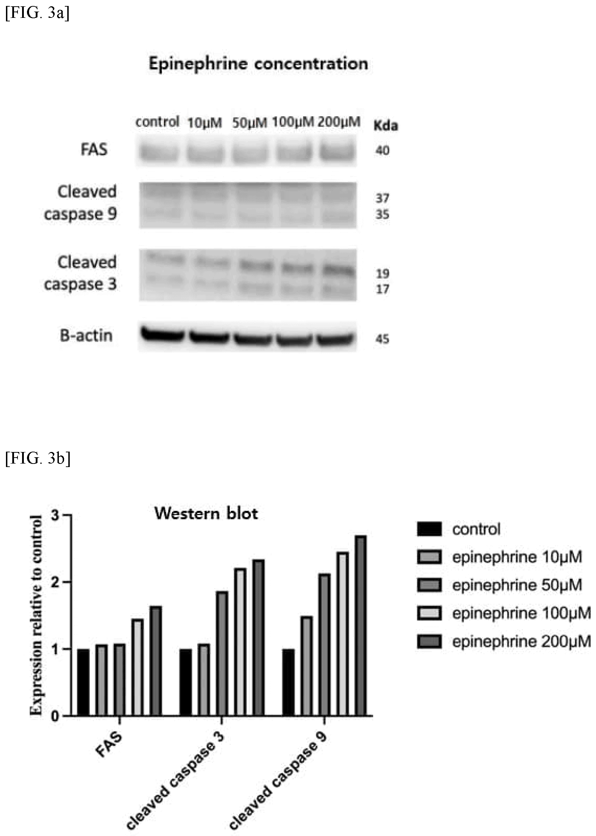

HUVEC cells cultured in culture dishes were lysed with a buffer containing 50 mM Tris (pH 8.0), 150 mM NaCl, 1% Nonidet p-40, 0.5% sodium deoxycholate (SDC), 0.1% sodium dodecyl sulfate (SDS), 1× protease inhibitor cocktail. Thereafter, the lysate was cooled on ice for 30 minutes and centrifuged at 14,000 g for 10 minutes, and the supernatant was separated. Quantification was performed using a bicinchoninic acid protein assay kit (Pierce Chemical, Rockford, IL, USA), and 30 µg of the protein solution was electrophoresed on a 10% SDS-polyacrylamide gel and then transferred to a nitrocellulose membrane. After the electrophoresed membrane was reacted with blocking milk for 1 hour, primary antibodies against cleaved caspase 3, cleaved caspase 9, and FAS were reacted at room temperature for 1 hour. The membrane was washed three times at 15-minute intervals with Tris buffer containing 0.1% Tween 20, and then reacted with secondary antibodies against the primary antibodies at room temperature for 1 hour, followed by detection using a chemiluminescent reagent (Amersham Life Science, Arlington Heights, IL, USA). The results are shown in FIG. 3a.

A protein extracted from HUVECs was used as a positive control. The expression results of FAS, cleaved caspase 3, and cleaved caspase 9 in HUVECs according to the concentration of epinephrine are shown in FIG. 3b.

As shown in FIGS. 3a and 3b, it was confirmed that the expression levels of FAS, cleaved caspase 3, and cleaved caspase 9 increased as the concentration of epinephrine increased.

### Example 3: Confirmation of Epinephrine-Induced DNA Laddering by Gel Electrophoresis

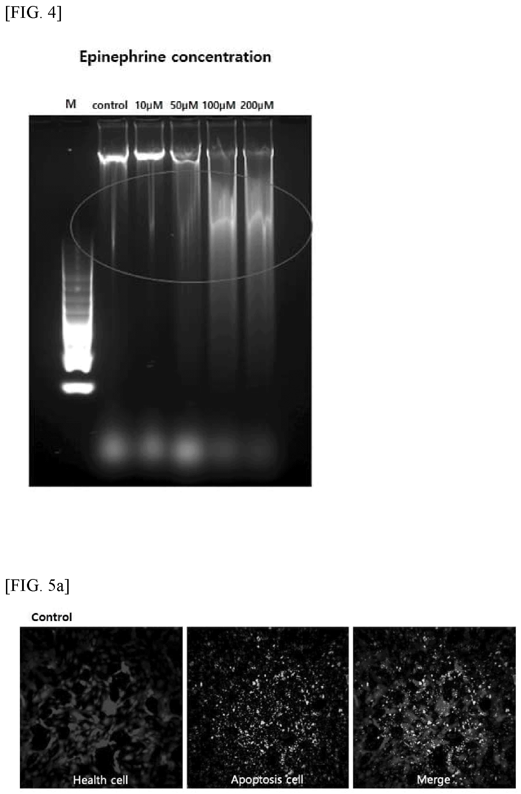

The characteristic DNA laddering observed during the electrophoresis procedure of Example 2 was confirmed, and the results are shown in FIG. 4. It can be seen that DNA fragmentation occurred as apoptosis of HUVECs was induced by epinephrine.

Referring to FIG. 4, it can be seen that, as the concentration of epinephrine increased, DNA fragmentation due to apoptosis of HUVECs increased, resulting in an increased migration rate of DNA.

### Example 4: Confirmation of Apoptosis of HUVECs According to Epinephrine Concentration by Confocal Fluorescence Microscopy

Using an apoptosis detection kit (blue, green) (ab176749), apoptotic cells and healthy cells can be simultaneously monitored. A PS sensor exhibits green fluorescence (Ex/Em = 490/525 nm) upon binding to PS on the cell membrane, thereby allowing confirmation of apoptosis. Healthy cells can be identified using CytoCalcein Violet 450 (Ex/Em = 405/450 nm), which is a dye for labeling the cytoplasm of living cells.

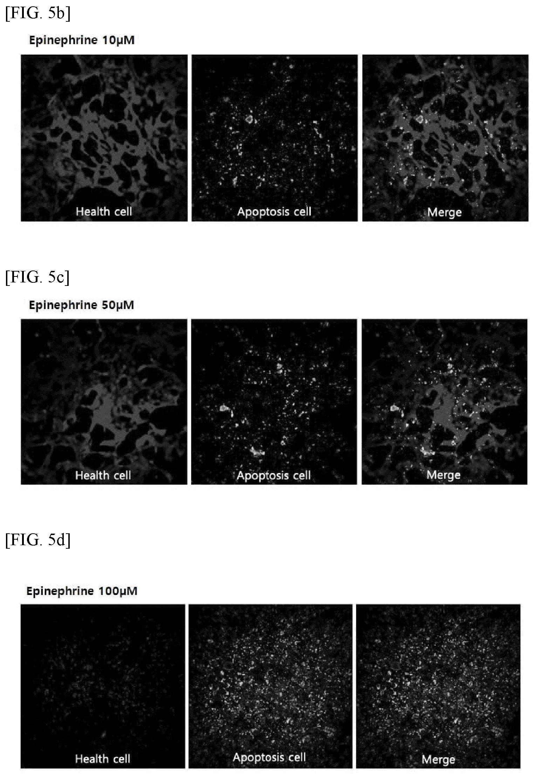

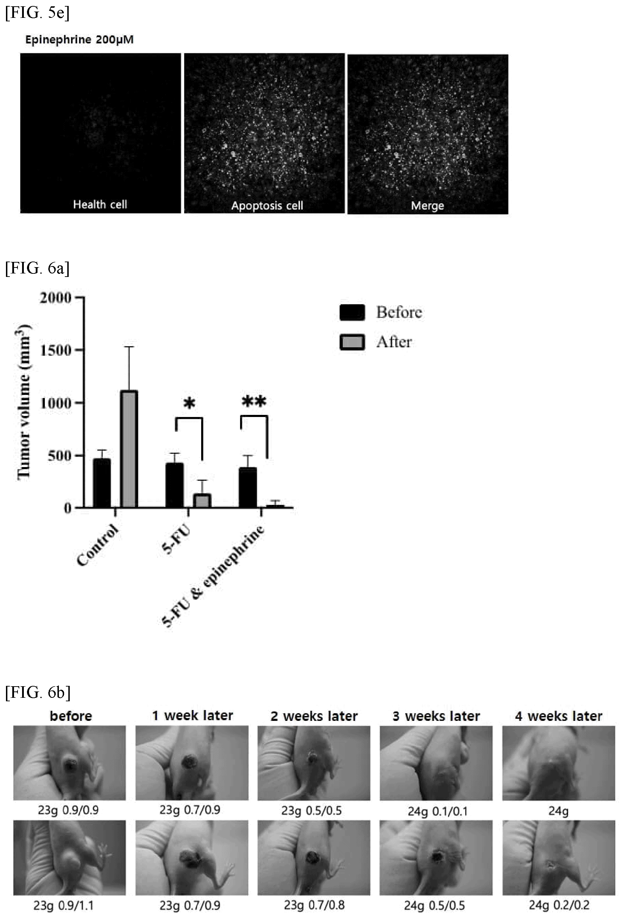

Vascular endothelial cells were cultured in a slide culture chamber, and epinephrine was administered to 5×10⁴ cells at various concentrations (0 to 200 µM) for 24 hours. Thereafter, the cells were washed one to two times by pipetting 100 µL of assay buffer up and down. The cells were then incubated with 200 µL of assay buffer containing Apopxin/CytoCalcein at room temperature for 30 to 60 minutes. After washing the cells twice with 100 µL of assay buffer, the buffer was replaced with 100 µL of fresh assay buffer, and the cells were transferred to the stage of a confocal microscope. After tilting the halogen lamp column backward and placing a slide on the stage, the analysis software was executed, and imaging parameters were set in the Acquire tab to XYZ mode, 1024×1024 pixels, 600 Hz, Zoom 1×, average 4, accumulation 1, and rotation 0. Thereafter, a sample was placed on the microscope and focused using fluorescence. After focusing, Beam Path Setting was configured in the order of selecting lasers to be used simultaneously, setting light intensity, selecting a PMT, selecting a pseudo color, and setting a detection band. When the live button was pressed, a preview scan was performed and the captured image was displayed in the right window. After checking the image while increasing the gain and intensity values, the final focal position was readjusted by turning the dial (Z position), and the image was captured, stored, and analyzed. The results are shown in FIGS. 5a to 5e.

Referring to FIGS. 5a to 5e, it can be seen that the number of healthy cells decreased and the number of apoptotic cells increased in proportion to the concentration of epinephrine.

### Example 5: In Vivo Experiment 1 for Confirming Vascular Endothelial Cell Toxicity of 5-FU and Epinephrine in Tumor Tissue

The animals used in the experiment were six 5-week-old male nude mice (Crj: BALB/c-nu/nu mice, male) produced by Orient, each having an average body weight of approximately 30 g. The mice were used after a one-week acclimation period at the research institute. Human gastric cancer cells (5×10⁶ cells/100 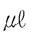 in PBS) were implanted into the dorsal subcutaneous fat layer of each nude mouse. The size of the tumors was periodically measured, and when the tumor diameter reached 1 cm, a drug administration experiment was conducted.

The experimental animals were divided into three groups. The first group was a control group and received only physiological saline. The second group received 0.2 cc of 5 mg of pH 8.4 5-FU alone. The third group received a total of 0.2 cc containing 5 mg of pH 8.4 5-FU and epinephrine at a concentration of 1:200,000. The compositions were injected adjacent to the tumor four times at one-week intervals. One month later, the major axis and minor axis of the tumor growing on the dorsal epidermis were measured, and the tumor size before and after drug administration was measured and compared as mean tumor volume = (major axis × minor axis²)/2 (mm³). The results are shown in FIG. 6a.

Referring to FIG. 6a, when alkaline 5-FU and epinephrine were administered together, the tumor size was significantly reduced as compared with the control group and the group administered alkaline 5-FU alone.

Additionally, photographs showing the tumor size of the third group before and after drug administration are presented in FIG. 6b. Referring to FIG. 6b, it can be confirmed that the tumor size significantly decreased over time.

### Example 6: In Vivo Experiment 2 for Confirming Vascular Endothelial Cell Toxicity of 5-FU and Epinephrine in Tumor Tissue

The animals used in the experiment were thirty-eight 5-week-old male nude mice (Crj: BALB/c-nu/nu mice, male) produced by Orient, each having an average body weight of approximately 30 g. The mice were used after a one-week acclimation period at the research institute. Human gastric cancer cells (5×10⁶ cells/100 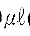 (PBS)) were implanted into the dorsal subcutaneous fat layer of each nude mouse. The size of the tumors was periodically measured, and when the tumor diameter reached 1 cm, a drug administration experiment was conducted.

When 5 mg of pH 8.4 5-FU was administered to 26 of the prepared 38 mice, six mice (about 23.1%) exhibited a rapid decrease in body weight or died.

When 0.2 cc of epinephrine at 1:200,0000 was injected together with 5 mg of pH 8.4 5-FU into the remaining 12 mice, only one mouse exhibited a decrease in body weight and died, confirming that the toxicity of the drug was reduced.

### Example 7: In Vivo Experiment for Confirming the Toxicity of 5-FU, Epinephrine, and L-Glucose to Vascular Endothelial Cells in Tumor Tissue

The animals used in the experiment were six 5-week-old male nude mice (Crj: BALB/c-nu/nu mice, male) produced by Orient, each having an average body weight of approximately 30 g. The mice were used after a one-week acclimation period at the research institute. Human gastric cancer cells (5×10⁶ cells/100 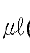 (PBS)) were implanted into the dorsal subcutaneous fat layer of each nude mouse. The size of the tumors was periodically measured, and when the tumor diameter reached 1cm, a drug administration experiment was conducted.

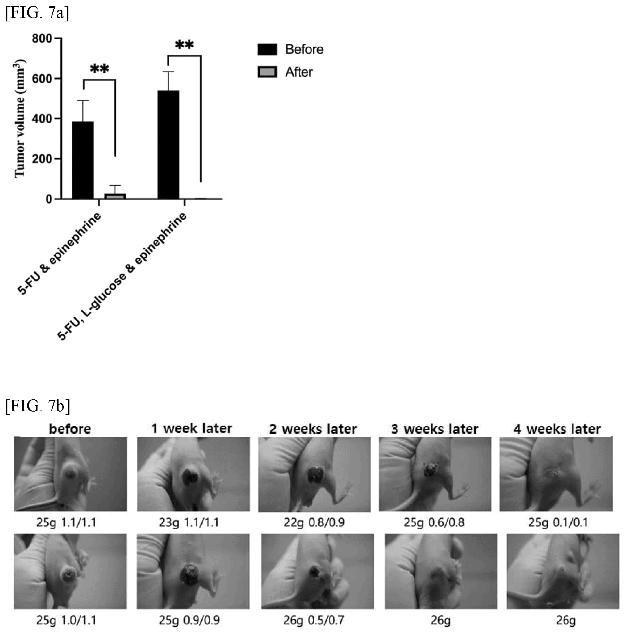

The experimental animals were divided into two groups. One group received a total of 0.2 cc containing 5 mg of pH 8.4 5-FU and epinephrine at 1:200,000, and the other group received a total of 0.2 cc containing 5 mg of pH 8.4 5-FU, epinephrine at 1:200,000, and 5% L-glucose. The compositions were injected adjacent to the tumor four times at one-week intervals. One month later, the major axis and minor axis of the tumor growing on the dorsal epidermis were measured, and the size before and after drug administration was measured and compared as mean tumor volume = (major axis × minor axis²)/2 (mm³). The results are shown in FIG. 7a.

Referring to FIG. 7a, when pH 8.4 5-FU, L-glucose, and epinephrine were injected, the tumor exhibited a nearly eradicated state as compared with the case in which 5-FU and epinephrine were injected.

Additionally, photographs showing the tumor size before and after drug administration in the case where pH 8.4 5-FU, L-glucose, and epinephrine were injected are shown in FIG. 7b.

Referring to FIG. 7b, when pH 8.4 5-FU, L-glucose, and epinephrine were injected, the size (volume) of the tumor was significantly reduced as compared with that before drug administration.

## Claims

1. A pharmaceutical composition for tumor vascular disruption, comprising alkaline 5-fluorouracil and epinephrine.

2. The pharmaceutical composition for tumor vascular disruption according to claim 1, wherein the alkalinity may have a pH of 8 to 9.

3. The pharmaceutical composition for tumor vascular disruption according to claim 1, wherein the alkaline 5-fluorouracil may be in a form in which 5-fluorouracil is dissolved in an alkaline solvent.

4. The pharmaceutical composition for tumor vascular disruption according to claim 1, wherein the alkaline 5-fluorouracil may cause death of vascular endothelial cells by increasing expression of thrombospondin-1 protein in tumor vascular endothelial cells.

5. The pharmaceutical composition for tumor vascular disruption according to claim 1, wherein the concentration of the alkaline 5-fluorouracil may be 0.1 to 600 mM based on the total composition.

6. The pharmaceutical composition for tumor vascular disruption according to claim 1, wherein the epinephrine may induce apoptosis of tumor vascular endothelial cells.

7. The pharmaceutical composition for tumor vascular disruption according to claim 1, wherein the concentration of the epinephrine may be 0.001 to 0.2 mg/mL based on the total composition.

8. The pharmaceutical composition for disrupting tumor blood vessels according to claim 1, further comprising one or more selected from glucose, a nitric oxide production inhibitor, bevacizumab, capric acid or a pharmaceutically acceptable salt thereof, and poloxamer.

9. The pharmaceutical composition for disrupting tumor blood vessels according to claim 8, wherein the nitric oxide production inhibitor comprises one or more selected from L-NMMA (N^{G}-Methyl-L-arginine), L-NAME (N^{G}-Nitro-L-arginine methyl ester), L-NA (Nitro Arginine), and 7NI (Nitroindazole).

10. The pharmaceutical composition for disrupting tumor blood vessels according to claim 1, further comprising one or more selected from cisplatin, carboplatin, cyclophosphamide, paclitaxel, tamoxifen, toremifene, fulvestrant, diindolylmethane, exemestane, raloxifene, an aromatase inhibitor, doxorubicin, oxaliplatin, vincristine, gemcitabine, anthracycline, taxane, irinotecan, docetaxel, and eribulin.

11. The pharmaceutical composition for tumor vascular disruption according to claim 1, wherein the tumor comprises a solid cancer.

12. The pharmaceutical composition for tumor vascular disruption according to claim 1, wherein the composition is a sustained-release formulation.

13. The pharmaceutical composition for tumor vascular disruption according to claim 1, wherein the composition is administered by local injection around a tumor.

14. The pharmaceutical composition for tumor vascular disruption according to claim 13, wherein the area around the tumor is a submucosal layer in which tumor blood vessels are located.

15. A local administration agent for a solid cancer, comprising the pharmaceutical composition for tumor vascular disruption according to claim 1.
